# EUROPEAN PATENT SPECIFICATION

(11) **EP 4 647 011 B1**
(45) Date of publication and mention of the grant of the patent: **22.04.2026**
(21) Application number: 25175343.0
(22) Date of filing: 09.05.2025
(51) Int. Cl.: A61B 5/367

(54) **VENTRICULAR TACHYCARDIA (VT) TARGET IDENTIFICATION BY SELECTIVE PACING**
ZIELIDENTIFIZIERUNG FÜR VENTRIKULÄRE TACHYKARDIE (VT) DURCH SELEKTIVES PACING
IDENTIFICATION DE CIBLE DE TACHYCARDIE VENTRICULAIRE (VT) PAR STIMULATION SÉLECTIVE

(30) Priority: 10.05.2024 US 202418661010
(43) Date of publication of application: 12.11.2025
(73) Proprietor: Biosense Webster (Israel) Ltd., 2066717 Yokneam (IL)
(72) Inventor: GOLDBERG, Stanislav, 2066717 Yokneam (IL); SZTEJNBERG, Dan, 2066717 Yokneam (IL); BEN NATAN, Alon, 2066717 Yokneam (IL); GREENBAUM, Lior, 2066717 Yokneam (IL); BEN-DOR, Amir, 2066717 Yokneam (IL); SHAPIRA, Einat, 2066717 Yokneam (IL); KEREN TAL, Dor Zeev, 2066717 Yokneam (IL); KARISI, Maor, 2066717 Yokneam (IL); ALON, Talia, 2066717 Yokneam (IL); SHALEV, Itai, 2066717 Yokneam (IL)
(74) Representative: Carpmaels & Ransford LLP

(56) References cited:
- US-A1- 2008 188 765
- US-A1- 2017 311 834
- US-A1- 2023 049 942

## Description

### FIELD OF THE DISCLOSURE

This disclosure relates generally to electrophysiological (EP) signals, and specifically to evaluation of electrical propagation in the heart.

### BACKGROUND OF THE DISCLOSURE

Estimation of electrophysiological signals to determine the location of ventricular arrhythmia was previously suggested in patent literature. For example, U.S. Patent 7,907,994 describes how ventricular tachycardia (VT) signals are induced in a living subject. Pace-mapped signals are then obtained from multiple points within the ventricle, and automatically compared numerically with the induced signals. Recognition of a high degree of cross-correlation between the induced signals and one or more of the pace-mapped signals identifies arrhythmogenic foci or pathways, which may then be ablated so that the arrhythmia becomes non-inducible.

As another example, U.S. Patent 10,891,728 describes a method for identifying an isthmus in a three-dimensional map of a cardiac cavity by means of a processing unit configured to perform the following steps: a) correlation between a set of stimulated points of the cardiac cavity, each stimulated point being represented by a set of signals that are obtained following surface electrocardiography (ECG), excluding ventricular tachycardia; b) identification of a watershed line based on the above correlation results and of the 3D coordinates of the stimulated points in the 3D map; and c) determination of the isthmus based on a 3D corridor substantially transverse to the watershed line.

US 2008/188765 A1 discloses that ventricular tachycardia signals are induced in a living subject. Pace-mapped signals are then obtained from multiple points within the ventricle, and automatically compared numerically with the induced signals. Recognition of a high degree of cross correlation between the induced signals and one or more of the pace-mapped signals identifies arrhythmogenic foci or pathways, which may then be ablated, so that the arrhythmia becomes non-inducible.

The present disclosure will be more fully understood from the following detailed description of the examples thereof, taken together with the drawings, in which:

### SUMMARY OF THE INVENTION

The invention is set out in the appended set of claims.

### BRIEF DESCRIPTION OF THE DRAWINGS

Fig. 1 is a schematic, pictorial illustration of a catheter-based electrophysiology (EP) pacing, mapping, and ablation system, according to an example of the present disclosure;
Fig. 2 illustrates schematically an EP map of a left ventricle superimposed with the large-area multi-electrode distal end assembly of Fig. 1, according to an example of the present disclosure;
Figs. 3A and 3B illustrate schematically methods to detect a candidate left ventricle arrhythmogenic location using the catheter of Fig. 1 and using area-level reference correlations (3A) or area-level intra-correlations (3B), according to two examples of the present disclosure;
Fig. 4 is a flow chart with a schematic description of a method to detect a left ventricle arrhythmogenic location using a large-area multi-electrode catheter, according to an example of the present disclosure; and
Fig. 5 is a flow chart with a detailed schematic description of a method to describe some steps of the method described in Fig. 4 to detect a left ventricle arrhythmogenic location using a large-area multi-electrode catheter, according to an example of the present disclosure.

### DETAILED DESCRIPTION OF EXAMPLES

### OVERVIEW

To characterize an arrhythmia of a cardiac chamber, such as a left ventricle (LV), a physician may use a catheter to pace (e.g., apply bipolar pacing signals between two adjacent catheter electrodes) at multiple LV tissue locations in search of suspected tissue pathways and circuits within the LV. During the pacing, if a transient arrhythmogenic event occurs, such as an ectopic beat, premature beat, or premature ventricle complex (PVC), the event may be recorded with a 12-lead ECG device showing an abnormal signal pattern.

Various methods can be used to recognize an induced arrhythmogenic event at a given ventricle location. For example, a physician may sample different areas of the ventricle and perform pattern matching (e.g., of 12-lead ECG waveforms) between acquired waveforms and stored pattern waveforms characteristic of the arrhythmia (e.g., VT), to identify correlations. A high degree of correlation indicates that the paced location is part of an arrhythmogenic tissue. As another example, the physician may compare only between waveforms acquired at different cardiac locations. In this case, a low degree of correlation indicates that at least one paced location is nearer to an arrhythmogenic tissue location.

At times, however, the physician has difficulty finding a location that induces a consistent correlation, whatever the correlation method. When this happens, it may be particularly difficult for the physician to determine in what direction to move the catheter to obtain a meaningful correlation.

One method to guide the physician to an arrhythmogenic ventricle area is described in U.S. Patent Application 18/19599, filed on May 11, 2023, titled "Spatial Correlation to Identify Ventricle Location of Pattern Matching," which is assigned to the assignee of the present application. This method includes comparing cardiac signals received from multiple locations within the ventricle with reference signals indicative of arrhythmia. Based on the comparison, a processor calculates a direction toward a VT target location that may demonstrate an increased correlation between the received signals and the reference signals, indicating said direction to a user.

Still, acquiring a large amount of good-quality data and finding meaningful correlations involves stabilizing the catheter at multiple tissue locations and performing multiple respective pacing. Such a workflow can be difficult to fulfill in practical clinical scenarios.

Some examples of the present disclosure that are described hereinafter rely on the benefit of using a large-area multi-electrode catheter placed in the LV to identify a VT target location with little or no catheter movement and minimal pacing steps during the clinical procedure.

In one example, a system is provided that includes an interface and a processor. The interface is configured to send signals to the multi-electrode catheter and receive cardiac signals, such as ECG signals from a 12-lead recorder, acquired in response to the sent signals. The processor is configured to apply pacing to the ventricle from multiple electrode locations over the circumference of an area of the catheter. The processor receives the cardiac signals acquired in response to the pacing and applies a correlation algorithm to calculate a plurality of correlations among the received signals. Based on the calculated correlations, the processor checks if the area includes an arrhythmogenic location identified with a predefined sufficient spatial resolution (e.g., to an area in the order of an area enclosed by adjacent catheter electrodes). If the resolution is insufficient, the processor defines a sub-area to pace. Then the processor applies subsequent pacing to a second region of the ventricle from multiple electrode locations over a circumference of the sub-area and receives subsequent cardiac signals in response to the subsequent pacing. The processor calculates subsequent correlations among the subsequent received signals. Based on the subsequent correlations, the processor ascertains whether the arrhythmogenic location is found in the sub-area. If an arrhythmogenic location is found with sufficient spatial resolution, the processor indicates the identified arrhythmogenic location to a user.

The disclosed method is applicable for use with any correlation calculation method, including both a reference correlation (high-correlation) searching method, such as described in U.S. Patent 7,907,994, and an intra-correlation (low-correlation) searching method, such as described in U.S. Patent 10,891,728.

The disclosed technique uses the observation that a bipolar pacing scheme produces a very localized indication of tissue type (e.g., one that covers a few mm² at most), which makes the search challenging. The authors found that using a large-area multi-electrode catheter expands the search area for indication of tissue type to an area as large as several hundred mm², depending on the catheter type. The disclosed technique covers such an area with a minimal number of bipolar pacing steps. By reducing the number of pacing events and the number of required moves of the catheter, the disclosed technique makes detecting VT location targets a much more efficient process.

In one example, a rectangular geometry (e.g., 12x30 mm²) catheter is used. After the physician places the catheter, an interface of a system used for the technique applies bipolar pacing to LV locations at the circumference (e.g., vertices) of the rectangle. The interface receives 12-lead ECG signals acquired in response to the pacing. Based on the spatial distribution of the correlations among the ECG signals, an analysis of these signals lets the processor determine if it is advised to narrow the search to a sub-area (e.g., a quadrant) without moving the catheter. In a second iteration, paced locations over the circumference of the sub-area are analyzed, which further narrows the search.

Typically, if an arrhythmogenic location exists under the catheter, two iterations are sufficient. However, an additional iteration that involves receiving signals from locations in the sub-area may be used to pinpoint the target location.

The selected search geometry may depend on catheter geometry. For example, when using a different catheter (e.g., a multi-arm catheter) it may be useful to search using a triangular grid or a specialized grid shape.

Finally, even if the arrhythmogenic (e.g., VT) target location is not found, a processor performing the iterative area-level correlation analysis can recommend a direction to move the catheter in search of the arrhythmogenic location, based on the spatial distribution of correlations. Such a direction may represent, for example, the direction of the steepest change in correlation.

### SYSTEM DESCRIPTION

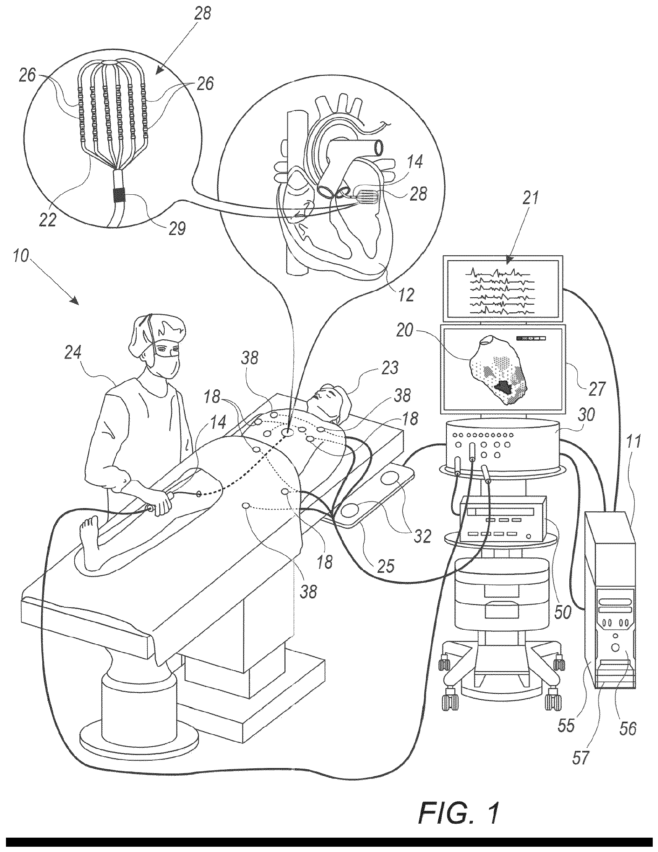

Fig. 1 is a schematic, pictorial illustration of a catheter-based electrophysiology (EP) pacing, mapping, and ablation system 10, according to an example of the present disclosure.

System 10 includes a catheter 14 which is percutaneously inserted via a sheath by physician 24 through the patient's vascular system into a left ventricle of heart 12. The catheter 14, illustrated herein by way of example, is configured for bipolar pacing. Physician 24 brings a distal end assembly 28 of catheter 14 into contact with the heart wall for pacing locations over a given area of heart 12 of a patient 23.

As seen, catheter 14 includes a large-area flat distal end assembly 28 that carries multiple electrodes 26 over a plurality of splines 22 and is configured to apply bipolar pacing signals. Catheter 14 may additionally include a position sensor 29, embedded in or near distal tip 28 on a shaft 46 of catheter 14, for tracking its position and orientation of distal end 28. Optionally, and preferably, position sensor 29 is a magnetic-based position sensor including three magnetic coils for sensing three-dimensional (3D) position and orientation.

Magnetic based position sensor 29 may be operated together with a location pad 25 that includes a plurality of magnetic coils 32 configured to generate magnetic fields in a predefined working volume. Real-time position of distal tip 28 of catheter 14 may be tracked based on magnetic fields generated with location pad 25 and sensed by magnetic based position sensor 29. Details of the magnetic based position sensing technology are described in U.S. Patent Nos. 5,5391,199; 5,443,489; 5,558,091; 6,172,499; 6,239,724; 6,332,089; 6,484,118; 6,618,612; 6,690,963; 6,788,967; 6,892,091.

System 10 includes one or more electrode patches 38 positioned for skin contact on patient 23 to establish a location reference for location pad 25 as well as impedance-based tracking of electrodes 26. For impedance-based tracking, electrical current is directed toward electrodes 26 and sensed at electrode skin patches 38 so that the location of each electrode can be triangulated via electrode patches 38. Details of the impedance-based location tracking technology are described in US Patent Nos. 7,536,218; 7,756,576; 7,848,787; 7,869,865; and 8,456,182.

A recorder 11 displays cardiac signals 21 (e.g., electrograms from a 12-lead ECG device acquired with body surface ECG electrodes 18). Recorder 11 may include pacing capability to pace the heart rhythm and/or may be electrically connected to a standalone pacer.

Patient interface unit (PIU) 30 is an interface configured to establish electrical communication between catheters, electrophysiological equipment, power supply and a workstation 55 to control system 10 operation and receive EP signals from the catheter or apply pacing signals. Electrophysiological equipment of system 10 may include, for example, multiple catheters, location pad 25, body surface ECG electrodes 18, electrode patches 38, an ablation energy generator 50, and recorder 11. Optionally, and preferably, PIU 30 additionally includes processing capability for implementing real-time computations of catheter locations and for performing ECG calculations.

Workstation 55 includes memory 57, a processor 56 unit with memory or with appropriate operating software loaded therein, and user interface capability. Workstation 55 may provide multiple functions, optionally including (i) modeling endocardial anatomy in three-dimensions (3D) and rendering the model or anatomical map 20 for display on a display device 27, (ii) displaying on display device 27 activation sequences (or other data) compiled from recorded cardiac signals 21 in representative visual indicia or imagery superimposed on the rendered anatomical map 20, (iii) displaying real-time location and orientation of multiple catheters within the heart chamber, and (iv) displaying sites of interest on display device 27, such as places where ablation energy has been applied. One commercial product embodying elements of system 10 is available as the CARTO^{™} 3 System, available from Biosense Webster, Inc., 31A Technology Drive, Irvine, CA 92618.

In some examples, processor 56 typically comprises a general-purpose computer, which is programmed in software to carry out the functions described herein. The software may be downloaded to the computer in electronic form, over a network, for example, or it may, alternatively or additionally, be provided and/or stored on non-transitory tangible media, such as magnetic, optical, or electronic memory.

System 10 may include an ablation energy generator 50 that is adapted to conduct ablative energy to one or more electrodes at a distal tip of a catheter configured for ablation. Energy produced by ablation energy generator 50 may include, but is not limited to, radiofrequency (RF) energy or pulsed-field ablation (PFA) energy, including monopolar or bipolar high-voltage DC pulses, to be used to effect irreversible electroporation (IRE), or combinations thereof.

For ablation, physician 24 similarly brings a distal end of an ablation catheter to a target site. One or more additional catheters can be inserted via the sheath. They may include a catheter for sensing intracardiac electrogram signals, a catheter dedicated for ablating and/or a catheter dedicated for both EP mapping and ablating.

This configuration of system 10 is shown by way of example, in order to illustrate certain problems that are addressed by examples of the present disclosure and to demonstrate the application of these examples in enhancing the performance of such a system. Examples of the present disclosure, however, are by no means limited to this specific sort of example system, and the principles described herein may similarly be applied to other sorts of medical systems. For example, other multi-electrode catheter types may be used, such as the OCTARAY^{™} catheter or a basket catheter.

### USING A LARGE-AREA MULTI-ELECTRODE CATHETER FOR PACING

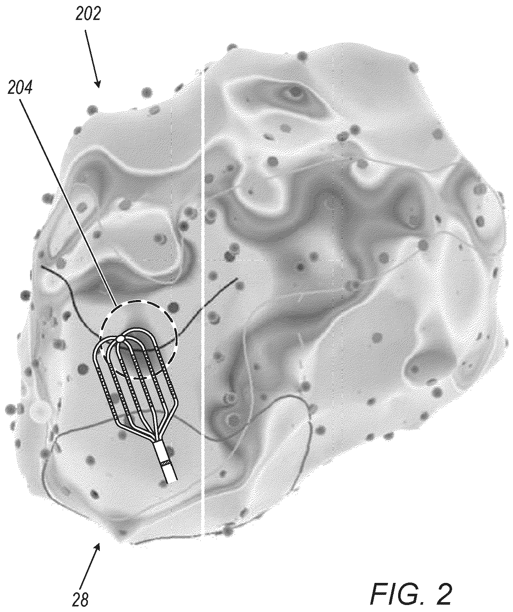

Fig. 2 illustrates schematically an EP map 202 of a left ventricle superimposed with the large-area multi-electrode distal end assembly 28 of Fig. 1, according to an example of the present disclosure. Fig. 2 also shows a possibly arrhythmogenic region 204. This region may include an isthmus that needs to be disconnected by ablation to eliminate a VT.

As seen, the area of distal end assembly 28 can be larger or comparable with a typical area of region 204. As a result, there is a good chance that an arrhythmogenic location is to be found under the catheter area. Even if the search area is larger than the catheter area, the disclosed technique covers such an expanded search area with a minimal number of bipolar pacing steps and catheter movements. This use of a large-area multi-electrode catheter for pacing makes detecting VT location targets, such as inside, or near, region 204, a much more efficient process.

As further seen, region 204 is not fully under distal end assembly 28 but within a capture range. This means that, in some cases, based on an area-level analysis of the correlation values, a processor can recommend a direction to move distal end assembly 28 to cover region 204 (i.e., to move up in Fig. 2).

### AREA-LEVEL ITERATIVE SEARCH OF VT TARGET LOCATION

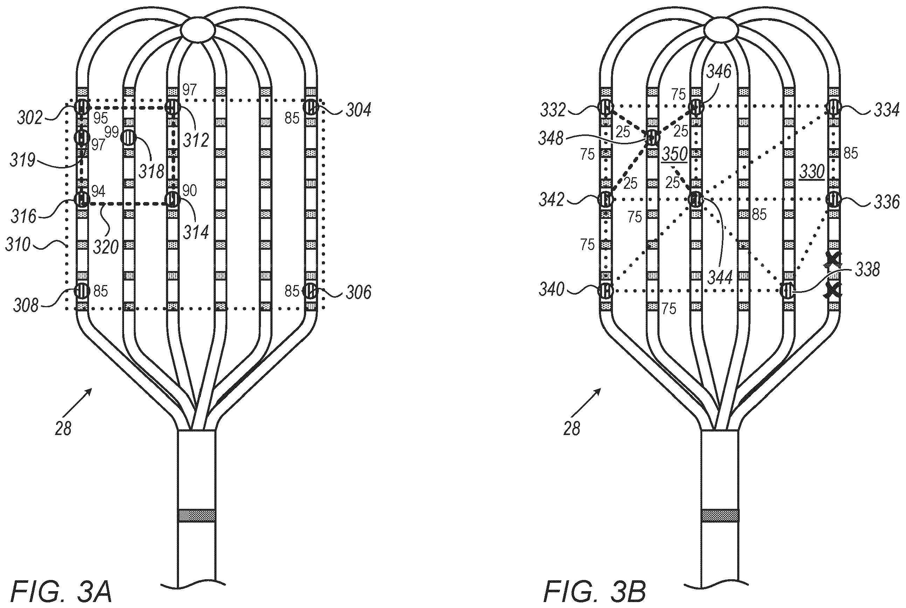

Figs. 3A and 3B schematically illustrate methods to detect a candidate left ventricle arrhythmogenic location using the catheter of Fig. 1 and using area-level reference correlations (3A) or area-level intra-correlations (3B), according to two examples of the present disclosure.

In Fig. 3A, ECG signals (not shown) are received in response to bipolar pacing at locations 302, 304, 306, and 308, over a circumference of catheter rectangular area 310. The respective reference correlation values (in percentage) of the locations are calculated to be 95, 85, 85, and 85. The higher reference correlation value at location 302 points to a candidate arrhythmogenic location or region being in the direction of location 302 (e.g., a top-left quadrant in a coordinate system with an origin in the center of rectangular 310).

In a second iteration of the disclosed method, ECG signals are received in response to bipolar pacing at locations 312, 314, and 316, over a circumference of assembly 28 sub-area 320. The respective reference correlation values (in percentage) of the locations in the vertices of sub-area 320 are calculated to be 95, 97, 90, and 94. The higher reference correlation value at locations 302 and 312 points to an arrhythmogenic location or region being in the direction of locations 302 and 312.

To pinpoint the target location, further ECG signals are received in response to bipolar pacing locations 318 and 319, yielding reference correlation values (in percentage) of 99 and 97, respectively.

Based on these results, the processor indicates location 318 as a target location (e.g., for ablation). Further mapping around arrhythmogenic location 318 (additional location not shown) can be done to determine the shape of the arrhythmogenic region (e.g., the shape of an isthmus).

In Fig. 3B, ECG signals are received in response to bipolar pacing at locations 332, 334, 336, 338, and 340, over a circumference of catheter area 330. Locations 336 and 338 are selected after the edge rectangular locations, marked by an "X", give unstable readings.

The respective intra-correlation values (in percentage) of ECG signals between paced locations (332-334), (332-334), (334-336), (336-338), (338-340), and (340-332) are calculated to be 75, 85, 90, 75, and 75, respectively. The lower intra-correlation values received when the left side locations are involved point to the arrhythmogenic location or region being on the left side of area 330.

In a second iteration of the disclosed method, ECG signals are received in response to bipolar pacing at locations 342, 344, and 346, over a circumference of catheter trapezoid shape area 350. Calculated intra-correlation values of 75% around the top left quadrant of the catheter point to the arrhythmogenic location or region possibly being in that quadrant.

To pinpoint the target location, further ECG signals are received in response to bipolar pacing location 348, yielding calculated intra-correlation values (in percentage) of 25. Based on these results, the processor indicates location 348 as a target location (e.g., for ablation). Again, further mapping around location 348 (not shown) can be done to determine the shape of the arrhythmogenic region (e.g., the shape of an isthmus).

As noted above, in case no target location is found under assembly 28, results comparable to those given in the first iteration of Figs 3A and 3B, but less obvious in a subsequent iteration, would still indicate the existence of a gradient of the correlation in the direction of the top left quadrant, prompting the user to move the catheter in such a direction in search of the arrhythmogenic region.

### METHOD OF AREA-LEVEL ITERATIVE SEARCH OF VT TARGET LOCATION

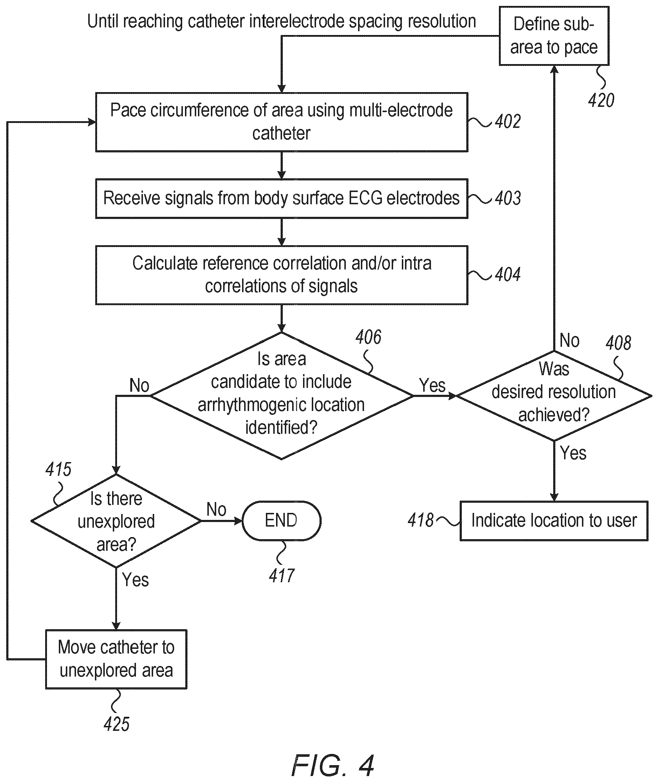

Fig. 4 is a flow chart with a schematic description of a method to detect a left ventricle arrhythmogenic location using a large-area multi-electrode catheter, according to an example of the present disclosure. The algorithm, according to the presented example, carries out a process that begins with system 10 pacing multiple locations at a left ventricle region using multi-electrode catheter 14, at a signal pacing step 402. Most, or all, of the locations are over the circumference of an area of catheter 14, as seen in Fig. 3.

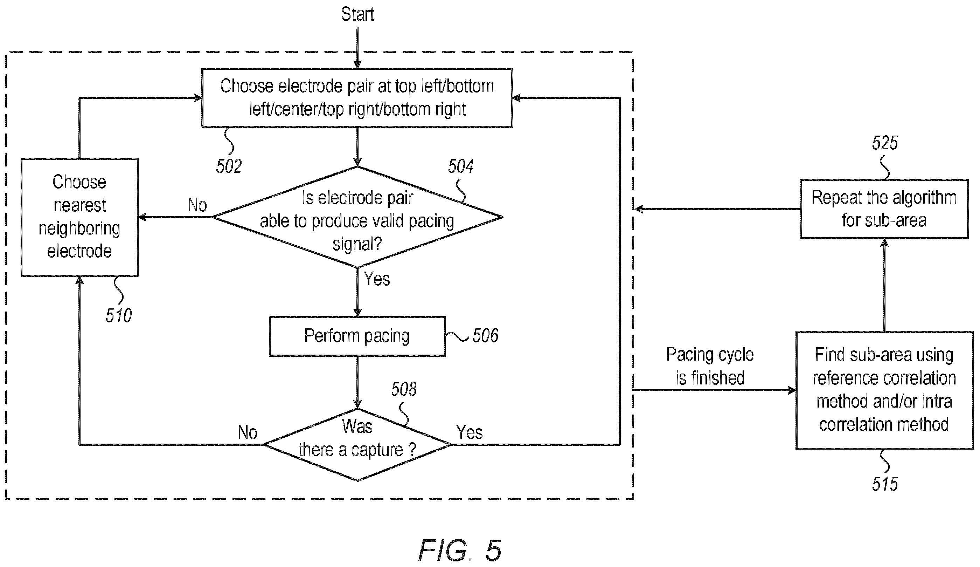

In response to the pacing, at a signal receiving step 403, system 10 receives respective ECG signals from body surface electrodes. A more detailed workflow of the signal application and acquisition steps 402-403 is provided in Fig. 5.

Next, the processor can correlate the received ECG waveforms with reference ECG waveforms indicative of VT (reference correlation method) and/or search for low correlation among the received waveforms (intra-correlation method), at waveform correlation step 404.

At identification checking step 406, processor 56 attempts to identify if the area of assembly 28 is candidate to include an arrhythmogenic location, based on the spatial distribution of the correlations.

If, at step 406, the processor identifies a candidate sub-area, the processor checks, at a required spatial resolution step 408, if the area identified to a required resolution (e.g., of a catheter inert electrode spacing of several millimeters).

If the answer is no, the processor uses the correlation information to determine a sub-area of the catheter to pace, at a sub-area defining step 420.

The processor then repeats step 402, adjusted to electrodes relevant to the sub-area.

If the answer is yes, at outputting step 418, the processor outputs an indication to a user of the arrhythmogenic location, for example by marking the location on EP map 202.

If, at checking step 406, the processor does not identify a candidate area for an arrhythmogenic location, the processor checks at a mapping checking step 415 if there is another LV area that was not explored (e.g., mapped) yet. If the answer is no, the process ends (step 417).

If the answer is yes, the processor instructs the user (e.g., physician 24) to move the catheter to the unexplored area, at a catheter moving instruction step 425. The processor then repeats step 402 in the new area.

In some cases, the processor can, based on the spatial distribution of the correlations, determine a direction can be defined toward such location, e.g., based on the presence of a consistent gradient of correlations, at direction checking step (not shown). If a direction is found, the physician moves the catheter in that direction and the process returns to step 402.

The flowchart of Fig. 4 is brought by way of example. For example, additional steps may be included, such as repeated acquisition inside the sub-area to EP map the arrhythmogenic region, which are omitted for simplicity.

Fig. 5 is a flow chart that schematically describes some steps of the method described in Fig. 4, at a more detailed level, to detect a left ventricle arrhythmogenic location using a large-area multi-electrode catheter, according to an example of the present disclosure.

Acquisition selection step 502 details an acquisition using catheter 14. With reference to Fig. 3, step 502 includes, at a first iteration, selecting one of locations 302, 304, 306, and 308 over a circumference of rectangular area 310 for bipolar pacing.

Next, at a signal quality check step 504, processor 56 checks if the selected electrode pair to be used for the bipolar pacing at the selected location can generate a valid signal. For example, in this step the system ensures that electrode-tissue contact is sufficient. This step may include checking that other criteria are met, as listed in the system definitions.

If the answer to step 504 is no, the processor replaces, at electrode replacing step 510, at least one of the electrodes of the pair with the nearest neighboring electrode, and the process returns to step 502.

If the answer to step 504 is yes, the process proceeds to pacing at a pacing step 506.

The processor checks if the ECG waveforms are received with the required quality, at capture checking step 508.

If capture quality is insufficient, the processor replaces, at electrode replacing step 510, at least one of the electrodes of the pair with the nearest neighboring electrode, and the process returns to step 502.

If the answer to step 508 is yes, the signal is saved, to be used in step 515, and the process returns to step 502 to pace from another of the remaining locations.

After all required signals are captured, analysis step 515 corresponds to step 403-406 of Fig. 4 where the processor runs a correlation method to identify an arrhythmogenic sub-area.

If a sub-area is found, the algorithm of Fig. 5 (e.g., steps 502-510) is applied to electrode pairs of the sub-area, in an analysis step 525 that corresponds to steps 402-404 of Fig. 4.

It will be appreciated that the present disclosure is not limited to what has been particularly shown and described hereinabove. Rather, the scope of the present disclosure includes both combinations and subcombinations of the various features described hereinabove, as well as variations and modifications thereof which would occur to persons skilled in the art upon reading the foregoing description.

## Claims

1. A system (10), comprising:
an interface (30) configured to send signals to a multi-electrode catheter (14) placed in a ventricle of a heart (12) of a patient and receive cardiac signals (21) acquired in response to the sent signals; and
a processor (56), which is configured to:
apply pacing to the ventricle from multiple electrode (26) locations over a circumference of an area (310) of the catheter (14);
receive cardiac signals (21) acquired in response to the pacing;
apply a correlation algorithm to the received signals (21) to calculate a plurality of correlations among the received signals;
based on the calculated correlations, check if the area (310) includes an arrhythmogenic location (318) identified with a predefined sufficient spatial resolution; if the resolution is insufficient, define a sub-area (320) to pace;
apply subsequent pacing to the ventricle from multiple electrode (26) locations over a circumference of the sub-area (320);
receive subsequent cardiac signals (21) in response to the subsequent pacing;
calculate subsequent correlations among the subsequent received signals (21);
based on the subsequent correlations, ascertain whether the arrhythmogenic location (318) is found in the sub-area (320); and
if an arrhythmogenic location (318) is found with the sufficient spatial resolution, indicate the identified arrhythmogenic location (318) to a user (24).

2. The system according to claim 1, wherein the processor (56) is configured to check the area (310) with sufficient spatial resolution by checking an area enclosed by a set of adjacent catheter electrodes (26).

3. The system according to any of claims 1 or 2, wherein, in response to failing to find an arrhythmogenic location (318), the processor (56) is further configured to, based on the calculated correlations, calculate, and indicate a direction to move the catheter (14).

4. The system according to any of claims 1 through 3, wherein the interface (30) is configured to receive cardiac signals (21) acquired in response to the sent signals by receiving ECG signals from body surface ECG electrodes (18).

5. The system according to any of claims 1 through 4, wherein the multi-electrode catheter (14) comprises a flat rectangular shape distal end assembly (28).

6. The system according to any of claims 1 through 4, wherein the multi-electrode catheter (14) comprises a multi-arm distal end assembly.

7. The system according to any of claims 1 through 6, wherein the processor (56) is configured to apply a correlation algorithm to the received signals (21) to calculate a plurality of correlations among the received signals (21) by correlating the signals with reference signals.

8. The system according to any of claims 1 through 7, wherein the processor (56) is configured to apply a correlation algorithm to the received signals (21) to calculate a plurality of correlations among the received signals by correlating between the signals (21).

9. The system according to any of claims 1 through 8, wherein the signals (21) acquired in response to the pacing are ECG signals acquired using body surface electrodes (18).

## Patentansprüche

1. System (10), umfassend:
eine Schnittstelle (30), die konfiguriert ist, um Signale an einen Multielektrodenkatheter (14) zu senden, der in einem Ventrikel eines Herzens (12) eines Patienten platziert ist, und Herzsignale (21) zu empfangen, die als Reaktion auf die gesendeten Signale erfasst werden; und
einen Prozessor (56), der konfiguriert ist zum:
Anlegen einer Stimulation an das Ventrikel von mehreren Elektroden (26)-positionen über einen Umfang eines Bereichs (310) des Katheters (14);
Empfangen von Herzsignalen (21), die als Reaktion auf die Stimulation erfasst werden;
Anwenden eines Korrelationsalgorithmus auf die empfangenen Signale (21), um eine Vielzahl von Korrelationen zwischen den empfangenen Signalen zu berechnen;
basierend auf den berechneten Korrelationen, Prüfen, ob der Bereich (310) einen Arrhythmiefokus (318) einschließt, der mit einer vorgegebenen ausreichenden räumlichen Auflösung identifiziert wurde; wenn die Auflösung unzureichend ist, Definieren eines zu stimulierenden Teilbereichs (320);
Anlegen einer nachfolgenden Stimulation an das Ventrikel von mehreren Elektroden (26)-positionen über einen Umfang des Teilbereichs (320);
Empfangen nachfolgender Herzsignale (21) als Reaktion auf die nachfolgende Stimulation;
Berechnen nachfolgender Korrelationen zwischen den nachfolgend empfangenen Signalen (21);
basierend auf den nachfolgenden Korrelationen, Bestimmen, ob sich der Arrhythmiefokus (318) in dem Teilbereich (320) befindet; und
wenn ein Arrhythmiefokus (318) mit der ausreichenden räumlichen Auflösung gefunden wird, Anzeigen des identifizierten Arrhythmiefokus (318) einem Benutzer (24).

2. System nach Anspruch 1, wobei der Prozessor (56) konfiguriert ist, um die Fläche (310) mit ausreichender räumlicher Auflösung zu überprüfen, indem er eine Fläche überprüft, die von einem Satz benachbarter Katheterelektroden (26) umschlossen ist.

3. System nach einem der Ansprüche 1 oder 2, wobei der Prozessor (56) als Reaktion auf das Versagen, einen Arrhythmiefokus (318) zu finden, ferner konfiguriert ist, um basierend auf den berechneten Korrelationen eine Richtung zum Bewegen des Katheters (14) zu berechnen und anzuzeigen.

4. System nach einem der Ansprüche 1 bis 3, wobei die Schnittstelle (30) konfiguriert ist, um Herzsignale (21) zu empfangen, die als Reaktion auf die gesendeten Signale erfasst werden, indem EKG-Signale von Körperoberflächen-EKG-Elektroden (18) empfangen werden.

5. System nach einem der Ansprüche 1 bis 4, wobei der Multielektrodenkatheter (14) eine distale Endanordnung (28) mit flacher Rechteckform umfasst.

6. System nach einem der Ansprüche 1 bis 4, wobei der Multielektrodenkatheter (14) eine mehrarmige distale Endanordnung umfasst.

7. System nach einem der Ansprüche 1 bis 6, wobei der Prozessor (56) konfiguriert ist, um einen Korrelationsalgorithmus auf die empfangenen Signale (21) anzuwenden, um eine Vielzahl von Korrelationen zwischen den empfangenen Signalen (21) durch Korrelieren der Signale mit Referenzsignalen zu berechnen.

8. System nach einem der Ansprüche 1 bis 7, wobei der Prozessor (56) konfiguriert ist, um einen Korrelationsalgorithmus auf die empfangenen Signale (21) anzuwenden, um eine Vielzahl von Korrelationen zwischen den empfangenen Signalen durch Korrelieren zwischen den Signalen (21) zu berechnen.

9. System nach einem der Ansprüche 1 bis 8, wobei die als Reaktion auf die Stimulation erfassten Signale (21) EKG-Signale sind, die unter Verwendung von Körperoberflächenelektroden (18) erfasst werden.

## Revendications

1. Système (10), comprenant :
une interface (30) configurée pour envoyer des signaux à un cathéter à électrodes multiples (14) placé dans un ventricule d'un cœur (12) d'un patient et recevoir des signaux cardiaques (21) acquis en réponse aux signaux envoyés ; et
un processeur (56) qui est configuré pour :
appliquer une stimulation au ventricule à partir de multiples localisations d'électrode (26) sur une circonférence d'une zone (310) du cathéter (14) ;
recevoir des signaux cardiaques (21) acquis en réponse à la stimulation ;
appliquer un algorithme de corrélation aux signaux reçus (21) pour calculer une pluralité de corrélations parmi les signaux reçus ;
en fonction des corrélations calculées, vérifier si la zone (310) comporte une localisation arythmogène (318) identifiée avec une résolution spatiale suffisante prédéfinie ; si la résolution est insuffisante, définir une sous-zone (320) à stimuler ;
appliquer une stimulation ultérieure au ventricule à partir de multiples localisations d'électrode (26) sur une circonférence d'une zone (320) ;
recevoir des signaux cardiaques (21) ultérieurs en réponse à la stimulation ultérieure ;
calculer des corrélations ultérieures parmi les signaux reçus (21) ultérieurs ;
en fonction des corrélations ultérieures, déterminer si la localisation arythmogène (318) est trouvée dans la sous-zone (320) ; et
si une localisation arythmogène (318) est trouvée avec la résolution spatiale suffisante, indiquer la localisation arythmogène (318) identifiée à un utilisateur (24).

2. Système selon la revendication 1, dans lequel le processeur (56) est configuré pour vérifier la zone (310) avec une résolution spatiale suffisante en vérifiant une zone délimitée par un ensemble d'électrodes de cathéter (26) adjacentes.

3. Système selon l'une quelconque des revendications 1 ou 2, dans lequel, en réponse au fait de ne pas trouver de localisation arythmogène (318), le processeur (56) est configuré en outre pour, en fonction des corrélations calculées, calculer, et indiquer une direction pour déplacer le cathéter (14).

4. Système selon l'une quelconque des revendications 1 à 3, dans lequel l'interface (30) est configurée pour recevoir des signaux cardiaques (21) acquis en réponse aux signaux envoyés en recevant des signaux ECG provenant d'électrodes ECG (18) de surface corporelle.

5. Système selon l'une quelconque des revendications 1 à 4, dans lequel le cathéter à électrodes multiples (14) comprend un ensemble extrémité distale (28) de forme rectangulaire plate.

6. Système selon l'une quelconque des revendications 1 à 4, dans lequel le cathéter à électrodes multiples (14) comprend un ensemble extrémité distale à bras multiples.

7. Système selon l'une quelconque des revendications 1 à 6, dans lequel le processeur (56) est configuré pour appliquer un algorithme de corrélation aux signaux reçus (21) pour calculer une pluralité de corrélations parmi les signaux reçus (21) en corrélant les signaux à des signaux de référence.

8. Système selon l'une quelconque des revendications 1 à 7, dans lequel le processeur (56) est configuré pour appliquer un algorithme de corrélation aux signaux reçus (21) pour calculer une pluralité de corrélations parmi les signaux reçus par corrélation entre les signaux (21).

9. Système selon l'une quelconque des revendications 1 à 8, dans lequel les signaux (21) acquis en réponse à la stimulation sont des signaux ECG acquis à l'aide d'électrodes (18) de surface corporelle.
